Europäisches Patentamt

European Patent Office

Office européen des brevets

(11) Publication number: **0 338 409 B1**

# EUROPEAN PATENT SPECIFICATION

(45) Date of publication of patent specification: **01.02.95** (51) Int. Cl.6: **C12N 9/72**, C12N 15/52, C12N 1/00

(21) Application number: **89106549.2**

(22) Date of filing: **13.04.89**

(54) **Low molecular weight derivatives of prourokinase and pharmaceutical compositions containing the same.**

(30) Priority: **18.04.88 GB 8809093**

(43) Date of publication of application:
**25.10.89 Bulletin  89/43**

(45) Publication of the grant of the patent:
**01.02.95 Bulletin  95/05**

(84) Designated Contracting States:
**AT BE CH DE ES FR GB GR IT LI NL SE**

(56) References cited:
**EP-A- 0 247 674**
**EP-A- 0 253 241**

(73) Proprietor: **FARMITALIA CARLO ERBA S.r.l.**
**Via Carlo Imbonati 24**
**I-20159 Milano (IT)**

(72) Inventor: **Brandazza, Anna**
**Via Cesare Battista, 45**
**Rivolta d'Adda (Cremona) (IT)**
Inventor: **Lansen, Jacqueline**
**Via Garigliano, 7**
**Milan (IT)**
Inventor: **Mazue, Guy**
**Via Don Gnocchi, 4**
**Milan (IT)**
Inventor: **Sarmientos, Paolo**
**Via Mosé Bianchi, 24**
**Milan (IT)**

(74) Representative: **Kolb, Helga, Dr. Dipl.-Chem. et al**
**Hoffmann, Eitle & Partner,**
**Patentanwälte,**
**Postfach 81 04 20**
**D-81904 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention is concerned with low molecular weight derivatives of prourokinase (single-chain urokinase-type plasminogen activator (scu-PA); and the preparation of such derivatives by recombinant DNA methods.

The derivatives provided by the invention exhibit interesting and improved pharmacological properties. In particular they are useful as agents having thrombolytic activity.

We have utilized site-directed mutagenesis techniques to modify the human gene coding for prourokinase (also called scu-PA) and we have expressed the mutated genes in recombinant strains of E.coli. The modifications were chosen with the objective of obtaining lower molecular weight derivatives.

The new molecular forms of prourokinase subject of the present invention, were then purified and their biochemical and biological properties have been characterized. They retain similar specific activities and selective clot lysis properties to the wild type prourokinase, however they have lost the prourokinase cell-binding domain and can therefore display an increased half-life in the circulation.

In addition, they can be produced more efficiently by rDNA technology and allow the preparation of thrombolytic agents at lower costs.

Introduction

The increasing knowledge of the molecular interactions that regulate physiological fibrinolysis has lead to important implications in the understanding of the mechanismus that dissolve blood clots, and in the development of new thrombolytic agents.

In the human fibrinolytic system a proenzyme, plasminogen, can be activated to the active enzyme, plasmin, by several types of plasminogen activators (Collen, D. and Lijnen, H.R. The fibrinolytic system in man. CRC Critical Reviews in oncology/hematology, 4.No. 3, 249, 1986; Verstraete, M. and Collen, D., Thrombolytic therapy in the Eighties Blood, 67, No. 6, 1529, 1986). Plasmin is the major protease responsible for the degradation of the fibrin component of a blood clot (Rakoczi, I., Wiman, B., and Collen, D., On the biological significance of the specific interactions between fibrin, plasminogen and antiplasmin, Biochim., Biophys. Acta, 540, 295, 1978; Robbins, K.C., Summaria, L., Hsieh, B., and Shah, R.S.; The peptide chains of human plasmin. Mechanism of activation of human plasminogen to plasmin, J. Biol. Chem., 242, 2333, 1967; Widman, B., Primary structure of the B chain of human plasmin, Eur. J. Biochem., 76, 129, 1977).

However, plasmin can also exert its proteolytic effect on several plasma proteins among which the components of the coagulation pathway fibrinogen, factor V and VII (Collen, D. and Lijnen, H.R., The bibrinolytic system in man, CRC Critical Reviews in oncology/hematology. 4, No. 3, 249, 1986; Verstraete, M. and Collen, D.: Thrombolytic therapy in the Eighties Blood, 67, No. 6, 1529, 1986; Wiman, B., B., Lijnen, H.R. and Collen, D., On the specific interaction between the lysine binding sites in plasmin and complementary sites in alfa2-antiplasmin and in fibrinogen, Biochim. Biophys. Acta, 579, 142, 1979).

Activation of plasminogen may occur at the systemic level, leading to circulating plasmin that is rapidly neutralized by alfa2-antiplasmin and thus not available for fibrinolysis (Collen, D. and Lijnen, H.R., The fibrinolytic system in man. CRC Critical Reviews in oncology/hematology, 4. No. 3, 249, 1986; Verstraete, M. and Collen, D., Thrombolytic therapy in the Eighties Blood, 67, No. 6, 1529, 1986). When the alfa2-antiplasmin level is markedly reduced, plasmin is less rapidly neutralized and can exert its proteolytic efforts not only on fibrin, but also on the blood coagulation proteins as described previously. Excessive lowering in the plasma concentrations of fibrinogen, factor V and VIII, together with the inhibitory effects exerted by some of the fibrinogen degradation products on the haemostatic process, on platelet aggregation and on fibrin polymerisation lead to haemostatic deficiency and subsequently to high bleeding risk (Latallo, Z.S., and Lpaciuk, S., New approach to thrombolytic therapy: the use of defibrase in connection with streptokinase, Thrombos. Diath. Haemouh., 56, 253, 1973; Totty, W.G., Gilula, L.A., Mc. Clennman, M., Ahmed, P., and Sherman L., Low dose intravascular fibrinolytic therapy, Radiology, 143, 59, 1982).On the other hand, activation of plasminogen may occur at the fibrin level (fibrinbound plasminogen activation) leading to fibrin-bound plasman (Collen, D. and Lijnen, H.R., The fibrinolytic system in man. CRC Critical Reviews in oncology/hematology, 4, No. 3, 249, 1986; Verstraete, M. and Collen, D., Thrombolytic therapy in the Eighties Blood, 67, No.6, 1529, 1986), which is, instead, not affected by alfa2-antiplasmin and cannot induce systemic fibrinogenolysis.

Urokinase and streptokinase, the most commonly used plasminogen activators in conventional thrombolytic therapy in man, have no specific activity for fibrin. Both compounds activate relatively indiscriminately either circulating or fibrin-bound plasminogen (Zamarron, C., Lijnen, H.R., Van Hoef, B., and Collen, D., Biological and thrombolytic properties of proenzyme and active forms of human urokinase. I. Fibrinolytic and Fibrinogenolytic properties in human plasma in vitro of urokinase obtained from human urine or by recombinant DNA technology, Throm. Haemostas., 52, 19, 1984; Samama, M., and Kher, A., Fibrinolytic and Antifibrinolytic Agents. Sem. Hop. Paris, 61, No. 20, 1423, 1985.). Therefore, the systemic haemostatic breakdown often encountered during treatment with streptokinase and urokinase and, consequently, the elevated bleeding risk have often hampered th widespread clinical use of these thrombolytic agents, despite their demonstrated clinical efficacy (Samama, M., and Kher, A., Fibrinolytic and Antifibrinolytic Agents, Sem. Hop. Paris, 61, No.20, 1423, 1985; Maizel, A.S., and Bookstein, J.J., Streptokinase, urokinase and tissue plasminogen activator, Pharmaco-kinetics, relative advantages and methods for maximizing rates and consistency of lysis, Cardiovasc. Intervent. Radiol., 9, 236, 1986; Bell, W.R., Streptokinase and urokinase in the treatment of pulmonary emboli, Thromb. Haemostas., 35, 57, 1976; Acar, J., Vahanian, A., Michel, P.L., Slama, M., Cormier, B. and Roger, V., Thrombolytic treatment in acute Myocardial Infarction, Seminars in Thromb. and Haemost., 13, No. 2, 186, 1987; Gruppo Italiano per lo studio della Streptochinasi nell'infarto miocardico (GISSI) Effectiveness of intravenous thrombolytic treatment in acute myocardial infarction, Lancet, 1, 397, 1986).

On the contrary, tissue-type plasminogen activator (t-PA) (Hoylaerts, M., Ryken, D.C., Lijnen, H.R. and Collen, D., Kinetics of activation of plasminogen by human tissue plasminogen activator; role of fibrin, J. Biol. Chem., 257, No. 6, 2912, 1982), and more recently prourokinase (proUK) (Husain, S.S., and Gurewhich, V., Purification and partial characterization of a single chain, high molecular weight form of urokinase in human urine. Arch. Biochem. Biophys., 220, 31, 1983), both natural proteins, were shown to be weak activators of the circulating plasminogen and, conversely, strong activators of the fibrin-bound plasminogen, without either systemic haemostatic breakdown or consumption of alfa2-antiplasmin and plasminogen, thus their clinical use may cause lesser bleeding risk.

The fibrin-specific thrombolytic activity of t-PA has been explained by its ability to bind fibrin through specific lysine binding sites, loacted in the triple-disulfide-bonded "kringle domains" of the molecule. Consequently, fibrin-bound plasminogen could be activated without significant haemostatic breakdown (Collen, D., and Lijnen, H.R., Tissue Plasminogen Activator, Mechanism of action and thrombolytic properties, Haemostasis, 16, No. 3, 25, 1986). On the other hand, proUK (also denominated single chain urokinase type plasminogen activator, scu-PA) does not bind to fibrin, however it displays fibrin-specific thrombolytic activity without systemic haemostatic consumption (Pannell, R., and Gurewich, V., Pro-Urokinase. A study of its stability in plasma and of the mechanism of its selective fibrinolytic effect, Blood, 67, 1215, 1986; Gurewich, V., and Pannell, R., Fibrin binding and zymogenic properties of single chain urokinase (Pro-Urokinase), Seminars in Thromb. and Haemostat., 13, No. 2, 146, 1987; Lijnen, H.R., Zamarron, C., Blaber, M., Winbler, M.E., and Collen, D., Activation of plasminogen by pro-urokinase. I. Mechanism J. Biol. Chem., 261, 1253, 1986).

Extensive research has been carried out on the isolation and characterization of the human genes encoding these proteins and subsequent production by recombinant DNA technology (Collen, D., Stassen, J.M., Marafino, B.J., Builder, JR.S., De Cock, F., Ogez, J., Tajiri, D., Pennica, D., Bennett, W.T., Salwa, T., and Hoyng, C.F. Biological properties of human tissue-type plasminogen activator obtained by expression of recombinant DNA in mammalian cells. J. Pharmacol. Exp. Ther., 231, No. 1, 146, 1984; Holmes, W.E., Pennica, D., Blaber, M., Rey, M.W., Gunzler, W.A., Steffens, G.J. and Heynecker, H.L., Cloning and expression of the gene for pro-urokinase in Escherichia coli, Biotechnology, 3, 923, 1985).

Recombinant t-PA was submitted to multicenter clinical trials in patients with acute myocardial infarction and was shown to be significantly more effective than streptokinase in the recanalization of obstructed coronary arteries (The European Cooperative Study Group for Recombinant Tissue-type Plasminogen Activator, Randomized trial of intravenous recombinant tissue-type plasminogen activator versus intravenous streptokinase in acute myocardia infarction, Lancet, 1, 842, 1985; Sheehan, F.H., Braunwald, E., Canner, P., Doodge, H.T., Gore, J., Van Natta P., Passamani, E.R., Williams, D.O., Zaret, B.. The effect of intravenous thrombolytic therapy on left ventricular function: a report on tissue-type plasminogen activator and streptokinase from the Thrombolysis in myocardial Infarction (TIMI Phase I), Circulation, 75, 4, 817, 1987).

Prourokinase is at present in phase II clinical trials and is thought to be, at least, as effective as t-PA in terms of thrombolytic activity and safety (Van de Werf, F., Nobuhara, M., and Collen, D. Coronary Thrombolysis with Human single-chain, urokinase type Plasminogen Activator (Pro-Urokinase) in Patients with Acute Myocardial Infarction. Annals of Internal Medecine, 104, 345, 1986; Van de Werf, F., Vanhaecke, J., De Geest, H., Verstraete, M., and Collen, D. Coronary Thrombolysis with recombinant single-chain urokinase type plasminogen activator in patients with acute myocardial infarction. Circulation, 74, No.5, 1066, 1986).

3

EP 0 338 409 B1

Background of the invention.

Urokinase-type plasminogen activators (u-PAs) are found in at least three different forms in human urine, plasma and conditioned culture medium from a variety of cell lines. The first form to be characterized as u-PA consisted of a fibrinolytically active polypeptide of 410 amino acids, with an apparent molecular weight of 54000 daltons, containing two disulfide-linked chains (Gunzler, W.A., Steffens, G.J., Oetting, F., Kim, SM.A., Frankus, E., and Flohe, L. The primary structure of high molecular mass urokinase from human urine. The complete amino acid sequence of the A chain. Hoppe-Seyler,s Z. Phisiol. Chem., 363, 1155, 1982).

The A-chain or light chain contains 157 amino acids and one triple disulfide-bonded "kringle" structure. This chain also contains a receptor binding domain for normal and neoplastic cells (monocytes, monocyte-like cells and A 431 epidermoid cells). The B chain or heavy chain (30000 daltons) consists of 253 amino acids and contain the catalytic domain.

This molecular form. of u-PA is generally termed urokinase (UK), two-chain urokinase (TC-UK) or high molecular weight urokinase (HMW-UK) (Gunzler, W.A., Steffens, G.J., Otting, F., Buze, G., and Fohe, L., Structural relationship between high and low molecular mass urokinase, Hoppe-Seyler's Z. Phisiol. Chem., 363, 133, 1982).

The second form of u-PA has a molecular weight of 33000 daltons and results from proteolytic degradation of the HMW form by either plasmin or trypsin. It is called low molecular weight urokinase (LMW-UK). Protein sequence determinations have revealed that LMW-UK is identical to HMW-UK except for the absence of the NH -terminal 135 amino acids that are specifically removed by the action of plasmin or trypsin (Steffens, G.J., Gunzler, W.A., Otting., F., Frankens, E., and Flohe, L. The complete amino acid sequence of low molecular weight urokinase from human urine. Hoppe-Seyler's Z. Physiol. Chem., 363, 1043, 1982).

The third form of u-PA identified is prourokinase (proUK), a single-chain (54000 daltons) form of urokinase which is therefore, also termed single chain urokinase type plasminogen activator (scu-PA).

All these molecular forms of urokinase type plasminogen activator have already been obtained by recombinant DNA technology (Zamarron, C., Lijnen, H.R., Van Hoef, B., and Collen, D., Biological and thrombolytic properties of proenzyme and active forms of human urokinase. I. Fibrinolytic and Fibrinogenolytic properties in human plasma in vitro of urokinase obtained from human urine or by recombinant DNA technology. Thromb. Haemostas., 52, 19, 1984; Holmes, W.E., Pennica, D., Blaber, M., Rey, M.W., Gunzler, W.A., Steffens, G.J. and Heynecker, H.L. Cloning and expression of the gene for pro-urokinase in Escherichia coli, Biotechnology, 3, 923, 1985; Gunzler, W.A., Henninger, W., Hennies, H.H., Otting, F., Schneider, J., Friderichs, E., Giertz, H., Flohe, L., Blaber, H. and Winkler M. Functional characterization of human urokinase produced in bacteria as compared to urokinase obtained from human urine. Haemostasis, 14, 60, 1984).

Starting with the human gene encoding for prourokinase, we have constructed, by site-specific mutagenesis, a set of modified genes encoding for new and original derivatives of single chain urokinase-type plasminogen activator. These derivatives are lower molecular weight analogs that lost sequence portions of different sizes in the first 162 amino acids.

The modified genes have been expressed in recombinant strains of E. coli, and the new recombinant products, in their single chain form, have been purified and characterized for their ability to specifically dissolve fibrin clots.

These new molecular entities, never found in nature before, constitute the object of the present invention. These single-chain low molecular height prourokinase derivatives have fibrin specific thrombolytic activity, and offer a practical alternative to native or recombinant proUK. Tests indicate that they have a longer half-life, resulting in prolonged pharmacological activity.

According to one aspect, the present invention provides a low molecular weight derivative of human prourokinase consisting of amino acid 1 to 10 of mature prourokinase joined to amino acids 136 to 411 of mature prourokinase.

Subject matter of the present invention is therefore a low molecular weight derivative of human pro-urokinase according to claim 1.

Embodiments thereof are subject matter of claims 2 to 5.

The invention also encompasses above derivatives further modified by way of amino acid deletion, insertion or substitution, or derivatives further comprising at least one additional amino acid.

The invention further comprises pharmaceutical compositions containing above derivatives, as well as the DNA sequences, vector and transformed host for preparing the same.

Mature prourokinase it to be understood as full-length prourokinase.

4

According to further aspects, the derivatives may include an additional methionine residue at the NH2 end of the polypeptide, or may further include at least one glycosidic side-chain. Additionally, the derivatives may include at least one aminoacidic sequence of any origin.

Derivatives according to a further aspect of the invention may be produced by treating the above derivatives with plasmin.

The derivatives of the present invention may be provided in the form of pharmaceutical composition in combination with a pharmaceutically acceptable carrier. Such composition are useful as thrombolytic agents.

Molecular characteristics of the new proUK deriviatives.

The new proUK derivatives were constructed by oligonucleotide directed mutagenesis. In particular, oligonucleotides were designed and synthesized to cause deletions of coding regions within the proUK gene. The principle of this method is to subclone the proUK gene into a vector which can be obtained in a single strand from, such as the phage vector M13. The recombinant single strand is annealed with a complementary synthetic oligodeoxyrebonucleotide containing the desired coding sequence and lacking the sequence to be deleted. DNA polymerase and ligase are then used to extend the new strand and to ligate it into a circular form. The newly created heteroduplex DNA is used to transform a cell line into which it can replicate and yield a progeny where the phage bearing the wild type gene or the gene with the desired deletion will segregate into two different moleculare species. The starting mutagenic oligonucleotides can then be used as probes to recognize the mutated genes.

The mutated genes were then inserted in E. coli expression vectors which could direct the synthesis of the new proUK derivatives. The recombinant molecules were then purified and characterized.

The new proUK derivatives can be summarized as follows:

a) a form called Δ125, (apparently molecular weight around 31 kd), that was obtained by deleting from the proUK gene the DNA sequence coding from amino acid 11 to amino acid 135. This proUK derivative, constituted of 286 amino acids, has lost the "kringle" domain while keeping the proteolytic domain. It has been isolated in its single chain form and can be converted by plasmin to its double-chain configuration. Δ125 is amidolytically active after treatment with plasmin.

b) a form called Δ140 (apparent molecular weight around 30 kd), that was obtained by deleting the first 140 amino acids from proUK. This derivative, constituted of 271 amino acids, has been isolated in its single chain configuration. Δ140 could be converted to the amidolytically active two-chain form by treatment with plasmin.

c) a form called Δ150 (apparent molecular weight around 29kd), that was obtained by deleting the first 150 amino acids from proUK. This derivative of 261 amino acids is amidolytically inactive in its single chain configuration. After treating with plasmin, Δ150 becomes fully active.

d) a form called Δ162 (apparent molecular weight around 27 kd), that was obtained by deleting the first 162 amino acids from proUK. This derivative, constituted of only 249 amino acids, is equivalent to the B chain urokinase without the first 4 amino acids.

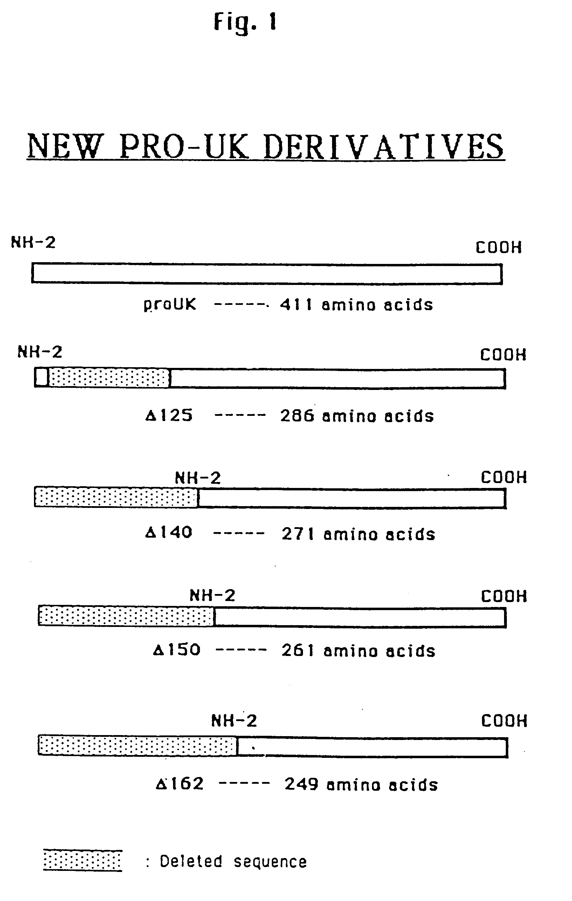

A schematic summary, describing these derivatives, is shown in Fig. 1. Their respective specific activities are shown in Table 1.

"In vitro" $^{125}$I-labeled fibrin clot lysis and fibrinogenolytic activity.

The relative fibrinolytic and fibrinogenolytic properties of some representative proUK derivatives of the invention were determined in an "in vitro" system consisting of $^{125}$I-labeled human plasma clots immersed in citrated human plasma as previously described (Matsuo, O., Ryken, D.C., and Collen, D. Comparison of the relative fibrinogenolytic, fibrinolytic and thrombolytic properties of tissue plasminogen activator and urokinase in vitro. Throm. Haemost., 45, 225, 1981). Wild-type proUK and human urinary two-chain urokinase were considered as reference products.

The $^{125}$I-labeled plasma clots were added to 2.0 ml aliquots of pooled plasma, counted, and incubated at 37°C in a water bath in the absence (control) or in the presence of 150 i.u./ml of each urokinase type plasminogen activator except for TC-UK which was studied at 50 i.u./ml. After a 4 hours incubation period, plasma samples were removed, the $^{125}$I-content measured and the percent of lysis determined from the solubilized radioisotope. In addition, plasma fibrinogen levels (Vermylen, C., De Vreker, R., and Verstraete, M. A rapid enzymatic method for assay of fibrinogen:fibrin polymerization time (FPT-test) Clin. Chim. Acta, 8, 418, 1963) were determined on each sample and expressed as percent of baseline value (control samples).

The stability of the urokinase type plasminogen activator (150 i.u./ml) for prouk and its LMW-derivatives; 50 i.u./ml for TC-UK) in plasma was measured after a 14 hours room temperatur pre-incubation period. After

5

this pre-incubation, freshly prepared $^{125}$I-labeled plasma clots were added to each mixture and placed at 37°C. Lysis rates were measured and calculated as above on triplicate samples.

The results obtained are summarized in Table 2. They indicate that all the low-molecular weight derivatives tested induce almost complete clot lysis activity without significant fibrinogenolysis. The low molecular weight proUK derivatives of the present invention display with highly efficient fibrin-specific clot lysis activity.

In addition, the low molecular derivatives of the invention maintained their clot lysis efficiency even after 24 h of incubation in human plasma indicating that these molecules, similarly to wild-type proUK but not to TC-UK are more stable in plasma.

Pharmacokinetic profile of the new proUK derivatives

The pharmacolkinetic profile of some representative proUK derivative of the present invention has been determined in normal rats after single intravenous injection of 80.000 IU/kg.

Urokinase-like antigen levels and fibrinolytic activity of the euglobulin fraction were determined before any treatment and after 0.5, 1, 2, 5, 10, 20, 30 minutes.

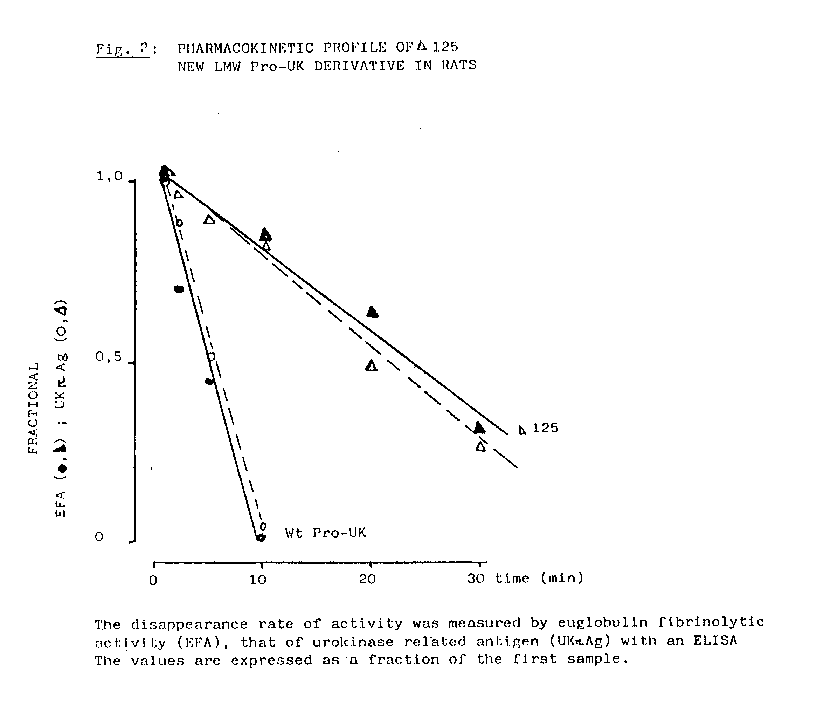

The results obtained are shown in Figure 2.

They indicate that the plasma disappearance rate of u-PA related antigen is much slower (initial t 1/2 of 25 min) for derivative Δ125 as compared with natural wild-type proUK (initial t 1/2 less than five min.).

In addition, the fibrinolytic activity of the euglobulin fraction is correlated to the plasma u-PA antigen level.

Interaction of the new proUK derivative with heparin.

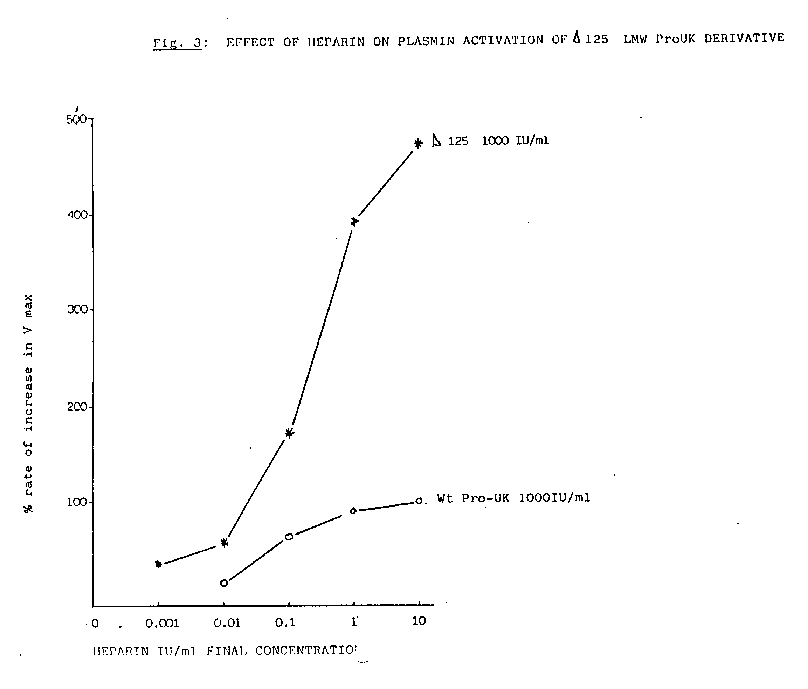

Heparin at pharmacological dosages conventionally encountered in plasma under anticoagulation therapy (between 0.01 and 1 IU/ml) was shown to potentiate "in vitro" at a highly significant level the rate of activation of some representative new LMW-derivative (Δ125) by plasmin (Figure 3).

Such effect was also observed but to a much lesser extent with wild type proUK.

Discussion and conclusions.

The observed amidolytic behaviour of the tested Δ125 derivatives of the present invention and of Δ140 and Δ150 indicated that any molecule which keeps the last 261 amino acids of prourokinase in a single-chain configuration could be fully amidolytically active upon plasmin activation by conversion in the two-chain configuration (Table 1).

Heparin, in concentrations which can be reached in plasma during therapeutic anticoagulation (0.01 - 1 IU/ml) concomitantly to thrombolytic therapy was shown to stimulate at a highly significant level the conversion of one of the new LMW derivatives of the invention (Δ125) in its two-chain configuration by plasmin. This effect was also obtained using wild-type proUK but to a much lesser extent (Figure 2).

On the other hand, almost complete lysis of $^{125}$I-labeled human fibrin clots were obtained with the new LMW derivatives without any consistent fibrinogenolysis which was not the case for two-chain urokinase.

In addition, this effect was maintained after at least 24 hours incubation of the new derivatives in plasma prior to the addition of the $^{125}$I-labeled human fibrin clot (Table 2). On the contrary, TC-UK clot lysis activity disappeared completely after long term pre-incubation in human plasma.

Finally, markedly longer half-life in the blood was observed after single intravenous injection of one of the new derivative (Δ125) of the invention to rats (Figure 3).

It may be concluded that:
- The new molecular weight derivatives of the present invention maintain the intrinsic amidolytic activity of wild-type proUK and may be fully activated in their amidolytically active two-chain form upon plasmin activation.
- Plasmain activation of the new molecular weight derivatives of the present invention and their subsequent conversion in their fully amidolytically active two chain configuration is highly stimulated by heparin. As heparin is always associated to thrombolytic therapy in clinical situation, this stimulatory effect on the new proUK derivatives of the present invention may lead to improved thrombolytic activity.
- The new molecular weight derivative of the present invention show more fibrin-specific clot lysis activity in comparison to TC-UK which is fully aspecific. Therefore, all proUK derivatives, which keep the amino acid sequence 151 to position 411, in a single-chain configuration can be regarded as improved thrombolytic agents over TC-UK with a less bleeding risk.

- In addition, the absence of the cell-binding domain of urokinase-type plasminogen activators located between residues 13-30 (Appella, E., Robinson, E.A., Ullrich, S.J., Stoppelli, M.P., Corti A., Cassani, G., and Blasi, F., The receptor binding sequence of urokinase, J. Biol. Chem., 262, No. 10, 4437, 1987) in the new proUK derivatives may prolong markedly their half-life in the circulation in comparison to wild-type proUK and TC-UK. Therefore, the fibrin-specific thrombolytic properties of the new low molecular weight pro-urokinase derivatives of the present invention together with the potential increase of this effect upon concomitant heparinotherapy and their potentially prolonged half-life in the circulation result in consistent therapeutic advantages of these molecules over those of two-chain urokinase and wild-type prourokinase.

Methods

Mutagenesis

The gene coding for human preprourokinase was cloned at the Weizmann Institute (Israel) according to published techniques Maniatis T., Fritsch E.F. and Sambrook J., Molecular clonging, A laboratory manual, Cold Spring Habour 1982). In order to carry out the desired mutagenesis, convenient restriction fragments were chosen and subcloned in M13 phage vectors (Zoller M.J. and Smith M. Oligonucleotide-directed mutagenesis using M13-derived vectors: an efficient and general procedure for the production of point mutations in any fragment of DNA, Nucleic Acid Research, 101, 6487, 1982).

Single strand forms of the recombinant phage vectors were grown according to the published methods. 20 ng of these M13 single strand DNAS were heated at 95°C in 10 mM Tris-HCl pH 7.5, 0.1 mM EDTA, 50 mM NaCl for 5 min and annealed to convenient oligonucleotides by stepwise cooling to room temperature. Successively, the following components were added: ATP to a final concentration of 0.4 mM; dCTP, dGTP, dTTP tp 0.12 mM, dATP to 0.04 mM; 1 unit of Klenow fragment of the E. coli DNA polymerase I and 0.5 units of T4 DNA ligase (Boehringer Mannheim). Final volume was 50 $\mu$l of 35 mM Tris-HCl pH 7.5, 0.1 mM EDTA, 6 mM MgCl$_2$, 0.006 mM DTT. After incubation for 12 hours at 15°C, the DNA was used to transfect E. coli JM 101 cells according to published procedures (Zoller M.J. and Smith M., Oligonucleotide-directed mutagenesis using M13 derived vectors; an efficient and general procedure for the production of point mutations in any fragment of DNA. Nucleic Acid Research, 101, 6487, 1982).

The oligonucleotides used to cause the desired deletions were radiolabeled using 150 $\mu$Ci ($\gamma$-32 P) ATP (new England Nuclear, 6000 Ci/mmol), in 70 mM Tris-HCl buffer pH 8, containing 10 mM MgCl$_2$, 5 mM DTT and 10 units of T4 Polynucleotide Kinase (Boehringer Mannheim) in a 50$\mu$l reaction mixture which was incubated for 30 min at 37°C. The labeled oligonucleotides were then used for plaque hybridization to the mutagenized phage DNAs.

Hybridization proceded overnight at 65°C in 10 mM Tris-HCl pH 8 containing 3xSSC, 0.1 % SDS, 10xDenhart's and 0.2 mg/ml denatured salmon sperm DNA. Nitrocellulose Filters were washed for 30 min in 0.4 SSC, 0.1 % SDS at 65°C with several changes and exposed overnight to x-ray films. Plaques indicating positive hybridization were selected for Sanger dideoxynucleotide sequencing using the Amersham M13 Sequencing Kit.

Using traditional expression plasmids (Maniatis T., Fritsch E.F. and Sambrook J., Molecular cloning, A laboratory manual, Cold Spring Harbour 1982), the mutated genes were then expressed in recombinant strains of E. coli which were then cultivated to allow production of the desired molecules.

Purification of the recombinant molecules

Following lysis of the bacteria, the recombinant products are found in the insoluble fraction. After washing the pellet in 10 mM Tris-HCl pH 8.5, 0.1 % Triton X100, the insoluble material is resuspended in 6M guanidine HCl, 0.1 M $\beta$-mercaptoethanol, 10 mM Tris-HCl pH 8.5. The suspension is shaken at 4°C for 16 hours and centrifuged to yield a supernatant which is then added, in a ratio of 1:25, to the following refolding buffer: 10 mM Tris HCl pH 8, 2.5 M Urea, 8 mM EDTA. The solution is held at 15°C for 24 hours. To reduce the ionic strength, the refolding solution is first concentrated 10 times by ultrafiltration and then diluted 4 times into a final buffer of 10 mM Tris HCl pH 7.8, 2.5 M Urea, 8 mM EDTA. The reactivated fibrinolytic agent is then purified by ion exchange chromatography on S-sepharose "fast flow" (Pharmacia) through a linear gradient of NaCl (0 to 1 M). The active fractions are collected, pooled, desalted by gel filtration on G-25 sephadex (Pharmacia) against 50 mM NH$_4$HCO$_3$ and freeze-dried. Final purity ranges from 80% to 98%.

As an alternative method, the derivative according to the invention can be expressed together with a signal peptide so that the derivative may be harvested from the supernatant.

Urokinase-type amidolytic activity

Amidolytic activity of TC-UK (Serono; batch No. 870402), proUK (Sero Institute, batch ODO1205100) and the low molecular weight derivatives of this invention (Δ125, Δ140, Δ150) were determined using the chromogenic substrate S-2444 (Glu-Gly-Arg-pNa; Kabi Vitrum). This assay is based upon the intrinsic amidolytic activity of tC-UK and the generation of this activity after the activation of the single-chain uokinase type plasminogen activators by plasmin. The amount of pNA generated upon reaction of the urokinase type plasminogen activators with the chromogenic substrate was monitored spectrophotometrically at 410 nm using a microplate reader (minireader II, Dynatech).

For plasmin activation, increasing concentrations of each plasminogen activator (range: from 1 ng/ml to 100 μg/ml) in Tris-HCl buffer pH 8.8 containing 0.05 M Tris, 0.038 M NaCl, 0.01% Tween 80 were incubated for 15 minutes at 37° C in the presence of plasmin (12.5 μg/ml). Appropriate controls (no plasmin activation) were included. After this incubation period, 20 μl of each activation mixture were added to 180 μl of a substrate solution containing 0.25 mg/ml of S-2444 and 100 KIU/ml of aprotinin in Tris-HCl buffer pH 8.8.

These mixtures were further incubated at 37° C for 30 minutes. The reaction was stopped by adding 50 μl of a 50% aqueous solution of acetic acid.

The same procedure was adopted for the International Reference Preparation Uokinase (n. 66/46) obtained from Dr.P.J. Gaffney, National Institute for Biological Standards and control (London, UK) and calibration curves were established.

For each product, the results in optical density were converted in IU/ml by using the above mentioned calibration curve.

Specific activities (IU/mg of protein) were calculated after measurements of the protein content of each urokinase type plasminogen activator preparation using the Bradford microassay from Bio-rad (standard II, BSA). S-2444 test and protein assays have been already desribed (Pannell, R., and Gurewich, V., Activation of plasminogen by single chain urokinase or by two chain urokinase. A demonstration that single-chain urokinase has a low catalytic activity (Prourokinase), Blood, 69, No. 1, 22, 1987; Bradford, M.M., A rapid and sensitive method for the quantitation of microgram quantities of proteins utilising the principle of protein-dye binding. Anal. Biochem., 72, 248, 1976).

Urokinase-type amidolytic activity in the presence of heparin

Wild type proUK (final concentration 1000 IU/ml) and the new proUK derivative Δ125 (final concentration 1000 IU/ml) in 0.05 M Tris-HCl buffer pH 7.4 containing 0.038 M NaCl and 0.01 % Tween 80 at 37°C was treated with plasmin (final concentration 10 mg/ml) in the absence or the presence of increasing concentration of heparin (0.001.10 IU/ml). At different time intervals(5,10,15 min) aliquots were removed and the generated two-chain urokinase-type amidolytic activity was measured with the chromogenic substrate S-2444 as previously described.

Clot lysis activity of urokinase-type plasminogen activators (Matsuo, O., Ryken, D.C., and Collen, D., Comparison of the relative fibrinogenolytic, fibrinolytic and thrombolytic properties of tissue plasminogen activator and urokinase in vitro. Thromb. Haemost., 45, 225, 1981).

Platelet-poor normal plasma (PPP) was obtained by centrifugation of a blood pool (collected in 10 mM trisodium citrate) a 4°C for 10 min. at 3000 g.

Aliquots of citrated human PPP (0.5 ml), containing human 125I-labeled fibrinogen (10,000 cpm, Amersham) were clotted by addition of $CaCl_2$ (final concentration 25 mM) and human thrombin (final concentration 2 NIH/ml). The mixtures were then aspirated in silicon tubings (internal diameter 4 mm) and alloved to clot for 30 min at 37 °C. Pieces of 1.5 cm length were cut. Clots were poured in Petri dishes and washed in 0.15 M NaCl for 5 min. at room temperature with several changes.

After radioisotope counting, the clots were immediately incubated at 37°C in 2 ml of PPP and 50 μl of a Tris-HCl 0.05M pH 7.4 buffer containing 0.038M NaCl and 0.01% Tween 80.

Urokinase type plasminogen activators solutions (150 IU/ml final concentration for Pro-UK, Δ125, Δ140, Δ150, and 50 IU/ml for TC-UK) or buffer (Tris-HCl 0.05M pH 7.4 buffer containing 0.038M NaCl and 0.01 % Tween 80) were added.

The extent of thrombolysis was calculated after 4 hours of incubation at 37°C from the amount of radioisotope released from the clot and recovered in the plasma medium in triplicate samples. The results were expressed as percent of baseline values (without fibrinolytic agent).

Stability of the urokinase type plasminogen activators. TC-UK (50 IU/ml), Pro-UK and its LMW-derivatives (150 IU/ml) were incubated in normal human citrated PPP for 24 hours at room temperature. After this, freshly prepared 125I-labeled human plasma clots were added and the extent of thrombolysis was measured as previously described (Matsuo, O., Ryken, D.C., and Collen, D., Comparison of the relative fibrinogenolytic, fibrinolytic and thrombolytic properties of tissue plasminogen activator and urokinase in vitro. Thromb. Haemost., 45, 225, 1981).

Plasma fibrinogen levels.

After 4 hours of incubation at 37°C of 125I-human plasma clots with each urokinase-type plasminogen activator, plasma aliquots (50 $\mu$l) were collected and their fibrinogen content measured according to the fibrinogen-fibrin polymerization test (Vermylen, C., De Vreker, R., and Verstraete, M., A rapid enzymatic method for assay of fibrinogen:fibrin polymerization time (FPT-test) Clin. Chim. Acta, 8, 418, 1963).

Pharmacokinetic study.

Normal rats (Sprague Dawley; Charles River), all males, weighing between 150-250 gr were used. All animals were fasten overnight and anesthesized with pentobarbital (50 mg/kg ip.) the day of the experiment. Wild-type proUK and Δ125 new LMW ProUK derivative were injected intravenously (tail vein) at a single dose of 80.000 IU/kg. Control animals were given saline. At different time intervals, 0 (before treatment), 0.5, 1, 2, 5, 10, 20, 30 min (after intravenous injection) blood samples were collected from the abdominal aorta and articoagulated with trisodium citrate (0-0.11 mole/l final concentration). For each product and time interval at least 3 animals were included. Blood samples were centrifuged (2000xg; 15 min; 4°C) and plasma was used for the measurement of the fibrinolytic activity of the euglobulan fraction according to the fibrin-plate method (Atrup T. and Muellertz, S., Arch. Biochem. Biophys., 40, 346-351, 1952) as well as the determination of urokinase-related antigen levels using an enzyme-linked immunosorbent assay (Elisa) calibrated against a wild-type proUK internal standard preparation (Grondahl, Hansen J., Agerlin N., Munkholm, Larsen P., Bach F., Nielsen L.S., Dombernowsky P. and Dano K., Sensitive and specific enzyme-linked immunosorbent assay for urokinase-type plasminogen activator and its application to plasma from patients with breast cancer, J. Lab. Clib. Med., 111, 1, 42-51, 1988).

## Table 1
### Urokinase-type amidolytic activity of low molecular weight pro-urokinase derivatives

| molecule | amidolytic activity (i.u./mg)* | |
|---|---|---|
| | without plasmin | with plasmin |
| Δ125 | undetectable | 96000 |
| Δ140 | undetectable | 85000 |
| Δ150 | undectectable | 105000 |
| pro-UK | undectectable | 98000 |
| TC-UK | 128000 | 128000 |

\* Urokinase-type amidolytic activity before and after plasmin treatment (12.5 $\mu$g/ml) was determined with the chromogenic substrate S-2444 (Pannell, R., and Gurewich, V.,

Activation of plasminogen by single-chain urokinase or by two chain urokinase. A. demonstration that single-chain urokinase has a low catalytic activity (Prourokinase), Blood, 69, No. 1, 22, 1987).

Pro-UK and its low molecular weight derivatives as well as human two chain urokinase (TC-UK) specific activities were determined upon calibration against the International Reference Preparation of Urokinase obtained from Dr. P.J. Gaffney, National Institute for Biological Standards and Control, London, UK.

Table 2

| Fibrinolytic and fibrinogenolytic activities of low molecular weight pro-urokinase derivatives | | | |
|---|---|---|---|
| Molecule | $^{125}$I-clot lysis activity in plasma (in %) | fibrinogen levels (in %) of baseline volumes | stability in plasma % of $^{125}$I-clot lysis activity after 24 h incubation |
| Δ125 | 95 | 85 | 90 |
| Δ140 | 85 | 90 | 90 |
| Δ150 | 100 | 50 | 85 |
| pro-UK | 100 | 85 | 80 |
| TC-UK | 100 | 10 | 0 |

**Claims**
**Claims for the following Contracting States : AT, BE, CH, LI, DE, FR, GB, IT, NL, SE**

1. A low molecular weight derivative of human pro-urokinase consisting of amino acid 1 to 10 of mature pro-urokinase joined to amino acids 136 to 411 of mature pro-urokinase.

2. A derivative according to Claim 1 further comprising an additional methionine residue at the amino terminal.

3. A derivative according to any preceding claim further comprising at least one glycosidic side chain.

4. A derivative according to any preceding claim constituting a protein as obtained by way of amino acid deletion, substitution or insertion, or further comprising at least one amino acidic sequence of any type.

5. A low molecular weight derivative of human pro-urokinase produced by treating a derivative according to any preceding claim with plasmin.

6. A pharmaceutical composition comprising a derivative according to any preceding claim and a pharmaceutically acceptable carrier.

7. A derivative according to any of claims 1 to 5 for use as a thrombolytic agent.

8. The use of a derivative according to any of claims 1 to 5 for the manufacture of a thrombolytic agent.

9. A DNA sequence coding for a derivative according to any of claims 1 to 5.

10. A process for producing a DNA sequence according to claim 9 comprising the step of conducting site specific mutagenesis on the human gene coding for pro-urokinase.

11. A process according to claim 10 wherein the site specific mutagenesis is carried out using a synthetic oligodeoxyribonucleotide lacking the sequence to be deleted and completing the sequence by the marker rescue technique.

12. An expression vector comprising a DNA sequence according to claim 9.

13. A host micro-organism transformed by a vector according to claim 12.

**Claims for the following Contracting States : ES, GR**

1. Process for the preparation of a low molecular weight derivative of human pro-urokinase consisting of amino acid 1 to 10 of mature pro-urokinase joined to amino acids 136 to 411 of mature pro-urokinase, characterized in that the proUK derivative is constructed by oligonucleotide-directed mutagenesis comprising the following steps: designing and synthesizing an oligonucleotide suitable to cause the desired deletion of coding regions within the proUK gene, subcloning the proUK gene into a vector which can be obtained in a single strand form, annealing the recombinant single strand with a complementary synthetic oligodeoxyribonucleotide containing the desired coding sequence and lacking the sequence to be deleted, using DNA polymerase and ligase to extend the new strand and to ligate it into a circular form, using the such created heteroduplex DNA to transform a cell line into which it can replicate and yield a progeny where the phage bearing the wild type gene or the gene with the desired deletion will segregate into two different molecular species, using the starting mutagenic oligonucleotide as probe to recognize the mutated gene, inserting the mutated gene in E. coli expression vectors which can direct the synthesis of the new proUK derivative and purifying the recombinant molecule .

2. Process of Claim 1, characterized in that a derivative is prepared which further comprises an additional methionine residue at the amino terminal.

3. Process of Claim 1, characterized in that a derivative is prepared comprising at least one glycosidic side chain.

4. Process of any one of Claims 1 to 3, characterized in that a derivative is prepared constituting a protein as obtained by way of amino acid deletion, substitution or insertion, or further comprising at least one amino acidic sequence of any type.

5. Process for the preparation of low molecular weight derivative of human pro-urokinase by treating a derivative as prepared according to any of the preceding claims with plasmin.

6. Process for preparation of a pharmaceutical composition comprising mixing a derivative as prepared according to any preceding claim and a pharmaceutically acceptable carrier.

7. The use of a derivative as prepared according to any of Claims 1 to 5 as a thrombolytic agent.

8. The use of a derivative as prepared according to any of Claims 1 to 5 for the manufacture of a thrombolytic agent.

9. A process for producing a DNA sequence coding for a derivative as prepared according to any of Claims 1 to 5, comprising the step of conducting site specific mutagenesis on the human gene coding for pro-urokinase.

10. A process according to Claim 9 wherein the site specific mutagenesis is carried out using a synthetic oligodeoxyribonucleotide lacking the sequence to be deleted and completing the sequence by the marker rescue technique.

11. Process for preparing a transformed host micro-organism comprising transforming a host micro-organism by an expression vector comprising a DNA sequence as prepared according to Claim 9.

12. A low molecular weight derivative of human pro-urokinase consisting of amino acid 1 to 10 of mature pro-urokinase joined to amino acids 136 to 411 of mature pro-urokinase.

**13.** A derivative according to Claim 12 further comprising an additonal methionine residue at the amino terminal

**14.** A derivative according to Claim 12 or 13 further comprising at least one glycosidic side chain.

**15.** A derivative according to any of Claims 12 to 14, constituting a protein as obtained by way of amino acid deletion, substitution or insertion, or further comprising at least one amino acidic sequence of any type.

**16.** A low molecular weight derivative of human pro-urokinase by treating a derivative prepared according to any of Claims 12 to 15 with plasmin.

**17.** A pharmaceutical composition comprising a derivative according to any of Claims 12 to 15 and a pharmaceutically acceptable carrier.

**18.** A derivative according to any of Claims 12 to 16 for use as a thrombolytic agent.

**19.** The use of a derivative according to any of Claims 12 to 16 for the manufacture of a thrombolytic agent.

**20.** A DNA sequence coding for a derivative as prepared according to any of Claims 12 to 16.

**21.** A process for producing a DNA sequence according to Claim 20 comprising the step of conducting site specific mutagenesis on the human gene coding for pro-urokinase.

**22.** A process according to Claim 21 wherein the site specific mutagenesis is carried out using a synthetic oligodeoxyribonucleotide lacking the sequence to be deleted and completing the sequence by the marker rescue technique.

**23.** An expression vector comprising a DNA sequence according to Claim 20.

**24.** A host micro-organism transformed by a vector according to Claim 23.

**Patentansprüche**

**Patentansprüche für folgende Vertragsstaaten : AT, BE, CH, LI, DE, FR, GB, IT, NL, SE**

**1.** Niedermolekulares Derivat von humaner Prourokinase, bestehend aus den Aminosäuren 1 bis 10 von reifer Prourokinase in Verbindung mit den Aminosäuren 136 bis 411 von reifer Prourokinase.

**2.** Derivat nach Anspruch 1, ferner umfassend einen zusätzlichen Methioninrest am Aminoende.

**3.** Derivat nach einem der vorstehenden Ansprüche, zusätzlich umfassend mindestens eine glycosidische Seitenkette.

**4.** Derivat nach einem der vorstehenden Ansprüche, das ein Protein darstellt, das durch Aminosäuredeletion, -substitution oder -insertion erhalten worden ist oder das zusätzlich mindestens eine Aminosäuresequenz eines beliebigen Typs umfaßt.

**5.** Niedermolekulares Derivat von humaner Prourokinase, gebildet durch Behandeln eines Derivats nach einem der vorstehenden Ansprüche mit Plasmin.

**6.** Pharmazeutische Zusammensetzung, umfassend ein Derivat nach einem der vorstehenden Ansprüche und einen pharmazeutisch verträglichen Träger.

**7.** Derivat nach einem der Ansprüche 1 bis 5 zur Verwendung als thrombolytisches Mittel.

**8.** Verwendung eines Derivats nach einem der Ansprüche 1 bis 5 zur Herstellung eines thrombolytischen Mittels.

**9.** DNA-Sequenz, die für ein Derivat nach einem der Ansprüche 1 bis 5 kodiert.

**10.** Verfahren zur Herstellung einer DNA-Sequenz nach Anspruch 9, umfassend die Stufe der Durchführung einer positionsspezifischen Mutagenese am humanen Gen, das für Prourokinase kodiert.

**11.** Verfahren nach Anspruch 10, wobei die positionsspezifische Mutagenese unter Verwendung eines synthetischen Oligodesoxyribonucleotids, dem die zu deletierende Sequenz fehlt, und durch Vervollständigung der Sequenz durch Markerreparaturtechnik durchgeführt wird.

**12.** Expressionsvektor, umfassend eine DNA-Sequenz nach Anspruch 9.

**13.** Wirtsmikroorganismus, transformiert durch einen Vektor nach Anspruch 12.

**Patentansprüche für folgende Vertragsstaaten : ES, GR**

**1.** Verfahren zur Herstellung eines niedermolakularen Derivats von humaner Prourokinase, das aus der Aminosäure 1 bis 10 von reifer Prourokinase in Verbindung mit den Aminosäuren 136 bis 411 von reifer Prourokinase besteht, dadurch gekennzeichnet, daß das proUK-Derivat konstruiert wird durch oligonucleotid-gerichtete Mutagenese, die folgende Stufen umfaßt: Entwerfen und Synthetisieren eines Oligonucleotids, das zur Herbeiführung der gewünschten Deletion von kodierenden Bereichen innerhalb des proUK-Gens geeignet ist, Subklonieren des proUK-Gens in einen Vektor, der in einzelsträngiger Form erhalten werden kann, Anelieren des rekombinanten Einzelstrangs mit einem komplementären synthetischen Oligodesoxyribonucleotid, das die gewünschte kodierende Sequenz enthält und dem die zu deletierende Sequenz fehlt, Verwendung von DNA-Polymerase und -Ligase zur Erweiterung des neuen Strangs und zur Verknüpfung zu einer Ringform, Verwendung der auf diese Weise erzeugten Heteroduplex-DNA zur Transformation einer Zellinie, in der sie replizieren und eine Nachkommenschaft erzeugen kann, wobei der Phage mit dem Wildtypgen oder mit dem Gen mit der gewünschten Deletion sich in zwei unterschiedliche Molekularspezies auftrennt, Verwenden des als Ausgangsprodukt verwendeten mutagenen Oligonucleotids als Sonde zur Erkennung des mutierten Gens, Inserieren des mutierten Gens in E. coli-Expressionsvektoren, die die Synthese des neuen proUK-Derivats dirigieren können, und Reinigen des rekombinanten Moleküls.

**2.** Verfahren nach Anspruch 1, dadurch gekennzeichnet, daß ein Derivat hergestellt wird, das zusätzlich einen weiteren Methioninrest am Aminoende umfaßt.

**3.** Verfahren nach Anspruch 1, dadurch gekennzeichnet, daß ein Derivat hergestellt wird, das mindestens eine glycosidische Seitenkette umfaßt.

**4.** Verfahren nach einem der Ansprüche 1 bis 3, dadurch gekennzeichnet, daß ein Derivat hergestellt wird, das ein Protein enthält, das durch Aminosäuredeletion, -substitution oder -insertion erhalten worden ist oder das zusätzlich mindestens eine Aminosäuresequenz eines beliebigen Typs umfaßt.

**5.** Verfahren zur Herstellung eines niedermolekularen Derivats von humaner Prourokinase durch Behandeln eines Derivats nach einem der vorstehenden Ansprüche mit Plasmin.

**6.** Verfahren zur Herstellung einer pharmazeutischen Zusammensetzung, umfassend das Vermischen eines nach einem der vorstehenden Ansprüche hergestellten Derivats und eines pharmazeutisch verträglichen Trägers.

**7.** Verwendung eines Derivats, das gemäß einem der Ansprüche 1 bis 5 hergestellt worden ist, als thrombolytisches Mittel.

**8.** Verwendung eines Derivats, das gemäß einem der Ansprüche 1 bis 5 hergestellt worden ist, zur Herstellung eines thrombolytischen Mittels.

**9.** Verfahren zur Herstellung einer DNA-Sequenz, die für ein nach einem der Ansprüche 1 bis 5 hergestelltes Derivat kodiert, umfassend die Stufe der Durchführung einer positionsspezifischen Mutagenese an dem für Prourokinase kodierenden humanen Gen.

**10.** Verfahren nach Anspruch 9, wobei die positionsspezifische Mutagenese unter Verwendung eines synthetischen Oligodesoxyribonucleotids, dem die zu deletierende Sequenz fehlt, und durch Vervollständigung der Sequenz durch die Markerreparaturtechnik durchgeführt wird.

**11.** Verfahren zur Herstellung eines transformierten Wirtsmikroorganismus, umfassend das Transformieren eines Wirtsmikroorganismus durch einen Expressionsvektor, der eine nach Anspruch 9 hergestellte DNA-Sequenz umfaßt.

**12.** Niedermolekulares Derivat von humaner Prourokinase, umfassend die Aminosäuren 1 bis 10 von reifer Prourokinase in Verbindung mit den Aminosäuren 136 bis 411 von reifer Prourokinase.

**13.** Derivat nach Anspruch 12, ferner umfassend einen zusätzlichen Methioninrest am Aminoende.

**14.** Derivat nach Anspruch 12 oder 13, ferner umfassend mindestens eine glycosidische Seitenkette.

**15.** Derivat nach einem der Ansprüche 12 bis 14, das ein Protein darstellt, das durch Aminosäuredeletion, -substitution oder -insertion erhalten worden ist oder zusätzlich mindestens eine Aminosäuresequenz eines beliebigen Typs umfaßt.

**16.** Niedermolekulares Derivat von humaner Prourokinase, erhalten durch Behandlung eines nach einem der Ansprüche 12 bis 15 hergestellten Derivats mit Plasmin.

**17.** Pharmazeutische Zusammensetzung, umfassend ein Derivat nach einem der Ansprüche 12 bis 15 und einen pharmazeutisch verträglichen Träger.

**18.** Derivat nach einem der Ansprüche 12 bis 16 zur Verwendung als thrombolytisches Mittel.

**19.** Verwendung eines Derivats nach einem der Ansprüche 12 bis 16 zur Herstellung eines thrombolytischen Mittels.

**20.** DNA-Sequenz, kodierend für ein nach einem der Ansprüche 12 bis 16 hergestelltes Derivat.

**21.** Verfahren zur Herstellung einer DNA-Sequenz nach Anspruch 20, umfassend die Stufe der Durchführung einer positionsspezifischen Mutagenese an dem für Prourokinase kodierenden humanen Gen.

**22.** Verfahren nach Anspruch 21, wobei die positionsspezifische Mutagenese unter Verwendung eines synthetischen Oligodesoxyribonucleotids, dem die zu deletierende Sequenz fehlt, und durch Vervollständigen der Sequenz durch die Markerreparaturtechnik durchgeführt wird.

**23.** Expressionsvektor, umfassend eine DNA-Sequenz nach Anspruch 20.

**24.** Wirtsmikroorganismus, transformiert mit einem Vektor nach Anspruch 23.

**Revendications**

**Revendications pour les Etats contractants suivants : AT, BE, CH, LI, DE, FR, GB, IT, NL, SE**

**1.** Dérivé de faible masse moléculaire de la pro-urokinase humaine, consistant en les acides aminés 1 à 10 de la pro-urokinase mature unis aux acides aminés 136 à 411 de la pro-urokinase mature.

**2.** Dérivé selon la revendication 1, comprenant en outre un résidu méthionine additionnel à l'extrémité terminale amino.

**3.** Dérivé selon l'une des revendications précédentes, comprenant en outre au moins une chaîne latérale glycosidique.

**4.** Dérivé selon l'une des revendications précédentes, constituant une protéine telle qu'obtenue à l'aide d'une délétion, d'une substitution ou d'une insertion d'acide aminé, ou comprenant en outre au moins une séquence d'acides aminés de n'importe quel type.

5. Dérivé de faible masse moléculaire de la pro-urokinase humaine, obtenu par traitement par la plasmine d'un dérivé tel que défini à l'une des revendications précédentes.

6. Composition pharmaceutique comprenant un dérivé tel que défini à l'une des revendications précédentes et un support pharmaceutiquement acceptable.

7. Dérivé selon l'une des revendications 1 à 5, pour utilisation en tant qu'agent thrombolytique.

8. Utilisation d'un dérivé tel que défini à l'une des revendications 1 à 5 pour la fabrication d'un agent thrombolytique.

9. Séquence d'ADN codant pour un dérivé tel que défini à l'une des revendications 1 à 5.

10. Procédé de fabrication d'une séquence d'ADN telle que définie à la revendication 9, comprenant l'étape consistant à effectuer une mutagénèse dirigée sur un site sur le gène humain codant pour la pro-urokinase.

11. Procédé selon la revendication 10, dans lequel on effectue la mutagénèse dirigée sur un site en utilisant un oligodésoxyribonucléotide synthétique dont la séquence devant être supprimée fait défaut et en complétant la séquence par la technique de sauvetage de marqueur.

12. Vecteur d'expression comprenant une séquence d'ADN telle que définie à la revendication 9.

13. Microorganisme hôte transformé par un vecteur tel que défini à la revendication 12.

**Revendications pour les Etats contractants suivants : ES, GR**

1. Procédé de préparation d'un dérivé de faible masse moléculaire de la pro-urokinase humaine, consistant en les acides aminés 1 à 10 de la pro-urokinase mature unis aux acides aminés 136 à 411 de la pro-urokinase mature, caractérisé par le fait que le dérivé de proUK est synthétisé par mutagénèse dirigée par un oligonucléotide comprenant les étapes suivantes :
   - concevoir et synthétiser un oligonucléotide approprié pour provoquer la délétion souhaitée des régions codantes au sein du gène de proUK ;
   - sous-cloner le gène de proUK dans un vecteur qui peut être obtenu sous une forme simple brin ;
   - amener sous forme bicaténaire le simple brin recombinant avec un oligodésoxyribonucléotide synthétique complémentaire contenant la séquence codante désirée et dans lequel la séquence devant être supprimée fait défaut ;
   - utiliser de l'ADN polymérase et de l'ADN ligase pour allonger le nouveau brin et pour le souder en une forme circulaire ;
   - utiliser l'ADN hétéroduplex ainsi créé pour transformer une lignée cellulaire dans laquelle il peut se répliquer et produire une descendance dans laquelle le phage portant le gène de type sauvage ou le gène ayant la délétion désirée va se séparer en deux espèces moléculaires différentes ;
   - utiliser l'oligonucléotide mutagène de départ en tant que sonde pour reconnaître le gène ayant subi une mutation ;
   - insérer le gène ayant subi une mutation dans des vecteurs d'expression d'E. Coli qui peuvent diriger la synthèse du nouveau dérivé de proUK ; et
   - purifier la molécule recombinante.

2. Procédé selon la revendication 1, caractérisé par le fait que l'on prépare un dérivé qui comprend en outre un résidu méthionine additionnel à l'extrémité terminale amino.

3. Procédé selon la revendication 1, caractérisé par le fait que l'on prépare un dérivé comprenant au moins une chaîne latérale glycosidique.

4. Procédé selon l'une des revendications 1 à 3, caractérisé par le fait que l'on prépare un dérivé constituant une protéine telle qu'obtenue à l'aide d'une délétion, substitution ou insertion d'acides aminés, ou comprenant en outre au moins une séquence d'acides aminés de n'importe quel type.

5. Procédé de préparation d'un dérivé de faible masse moléculaire de la pro-urokinase humaine par traitement par la plasmine d'un dérivé tel que préparé conformément à l'une des revendications précédentes.

6. Procédé de préparation d'une composition pharmaceutique comprenant le mélange d'un dérivé tel que préparé conformément à l'une des revendications précédentes, et d'un support pharmaceutiquement acceptable.

7. Utilisation d'un dérivé tel que préparé conformément à l'une des revendications 1 à 5 en tant qu'agent thrombolytique.

8. Utilisation d'un dérivé tel que préparé conformément à l'une des revendications 1 à 5, pour la fabrication d'un agent thrombolytique.

9. Procédé de fabrication d'une séquence d'ADN codant pour un dérivé tel que préparé conformément à l'une des revendications 1 à 5, comprenant l'étape consistant à effectuer une mutagénèse dirigée sur un site sur le gène humain codant pour la pro-urokinase.

10. Procédé selon la revendication 9, dans lequel on effectue la mutagénèse dirigée sur un site en utilisant un oligodésoxyribonucléotide synthétique dont la séquence devant être supprimée fait défaut et en complétant la séquence par la technique de sauvetage de marqueur.

11. Procédé de préparation d'un microorganisme hôte transformé, comprenant la transformation d'un microorganisme hôte par un vecteur d'expression comprenant un séquence d'ADN telle que préparée conformément à la revendication 9.

12. Dérivé de faible masse moléculaire de la pro-urokinase humaine, consistant en les acides aminés 1 à 10 de la pro-urokinase mature unis aux acides aminés 136 à 411 de la pro-urokinase mature.

13. Dérivé selon la revendication 12, comprenant en outre un résidu méthionine additionnel à l'extrémité terminale amino.

14. Dérivé selon la revendication 12 ou 13, comprenant en outre au moins une chaîne latérale glycosidique.

15. Dérivé selon l'une des revendications 12 à 14, constituant une protéine telle qu'obtenue à l'aide d'une délétion, substitution ou insertion d'acides aminés, ou comprenant en outre au moins une séquence d'acides aminés de n'importe quel type.

16. Dérivé de faible masse moléculaire de la pro-urokinase humaine obtenu par traitement par la plasmine d'un dérivé préparé conformément à l'une des revendications 12 à 15.

17. Composition pharmaceutique comprenant un dérivé tel que défini à l'une des revendications 12 à 15 et un support pharmaceutiquement acceptable.

18. Dérivé selon l'une des revendications 12 à 16 pour utilisation en tant qu'agent thrombolytique.

19. Utilisation d'un dérivé tel que défini à l'une des revendications 12 à 16, pour la fabrication d'un agent thrombolytique.

20. Séquence d'ADN codant pour un dérivé tel que préparé conformément à l'une des revendications 12 à 16.

21. Procédé de fabrication d'une séquence d'ADN telle que définie à la revendication 20, comprenant l'étape consistant à effectuer une mutagénèse dirigée sur un site sur le gène humain codant pour la pro-urokinase.

22. Procédé selon la revendication 21, dans lequel on effectue la mutagénèse dirigée sur un site en utilisant un oligodésoxyribonucléotide synthétique dans lequel la séquence devant être supprimée fait défaut et en complétant la séquence par la technique de sauvetage de marqueur.

**23.** Vecteur d'expression comprenant une séquence d'ADN telle que définie à la revendication 20.

**24.** Microorganisme hôte transformé par un vecteur tel que défini à la revendication 23.

# Fig. 1

# NEW PRO-UK DERIVATIVES

NH-2                                                                 COOH

proUK  -----. 411 amino acids

NH-2                                                                 COOH

Δ125  ----- 286 amino acids

NH-2                    COOH

Δ140  ----- 271 amino acids

NH-2                    COOH

Δ150  ----- 261 amino acids

NH-2                    COOH

Δ162  ----- 249 amino acids

: Deleted sequence

18

Fig. 2: PHARMACOKINETIC PROFILE OF Δ 125
NEW LMW Pro-UK DERIVATIVE IN RATS

The disappearance rate of activity was measured by euglobulin fibrinolytic
activity (EFA), that of urokinase related antigen (UKπAg) with an ELISA
The values are expressed as a fraction of the first sample.

EP 0 338 409 B1

Fig. 3: EFFECT OF HEPARIN ON PLASMIN ACTIVATION OF Δ 125 LMW ProUK DERIVATIVE